(19)

**Europäisches Patentamt**
**European Patent Office**
**Office européen des brevets**

(11) **EP 1 928 597 B1**

(12) **EUROPÄISCHE PATENTSCHRIFT**

(45) Veröffentlichungstag und Bekanntmachung des Hinweises auf die Patenterteilung:
**03.06.2009 Patentblatt 2009/23**

(21) Anmeldenummer: **06775208.9**

(22) Anmeldetag: **25.09.2006**

(51) Int Cl.:
***B01J 19/08*** *(2006.01)* ***A61K 9/51*** *(2006.01)*

(86) Internationale Anmeldenummer:
**PCT/CH2006/000517**

(87) Internationale Veröffentlichungsnummer:
**WO 2007/036060 (05.04.2007 Gazette 2007/14)**

(54) **VERFAHREN ZUR ANLAGERUNG VON NANOPARTIKELN AN SUBSTRATPARTIKEL**

METHOD FOR THE ATTACHMENT OF NANOPARTICLES TO SUBSTRATE PARTICLES

PROCEDE POUR DEPOSER DES NANOPARTICULES SUR DES PARTICULES DE SUBSTRAT

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.09.2005 US 721209 P**
**23.12.2005 CH 20582005**

(43) Veröffentlichungstag der Anmeldung:
**11.06.2008 Patentblatt 2008/24**

(73) Patentinhaber: **ETH Zürich**
**8092 Zürich (CH)**

(72) Erfinder:
• **SPILLMANN, Adrian**
**CH-8057 Zürich (CH)**

• **SONNENFELD, Axel**
**CH-8003 Zürich (CH)**
• **RUDOLF VON ROHR, Philipp**
**CH-4132 Muttenz / BL (CH)**

(74) Vertreter: **Bremi, Tobias Hans et al**
**Isler & Pedrazzini AG**
**Gotthardstrasse 53**
**Postfach 1772**
**8027 Zürich (CH)**

(56) Entgegenhaltungen:
**WO-A2-20/04052778      CN-A- 1 436 623**
**US-A1- 2003 138 368      US-A1- 2004 065 170**

EP 1 928 597 B1

**Beschreibung**

TECHNISCHES GEBIET

Die vorliegende Erfindung betrifft ein Verfahren zur Anlagerung von Nanopartikeln an Substratpartikel.

STAND DER TECHNIK

Die Verarbeitung von Feststoffen ist ein weit verbreiteter Vorgang, welcher in verschiedensten Industriezweigen Anwendung findet. Dies gilt insbesondere für die pharmazeutische Industrie, wo typischerweise bis zu 80% aller Zwischenstufen und praktisch alle Endstufen als Feststoffe isoliert und weiterverarbeitet respektive in pharmazeutische Darreichungsformen formuliert werden. Austragen, Dosieren und Mischen sind Abläufe, welche eine Bewegung des Feststoffschüttguts voraussetzen, wobei die Interaktionskräfte zwischen den Partikeln und den Partikeln und der Apparatewand eine wichtige Rolle spielen Bei feinkörnigen Stoffen (< 20$\mu$m) dominieren die Van-der-Waals Kräfte gegenüber der Gravitation. Dies hat zur Folge, dass diese Stoffe nur beschränkt oder gar nicht fliessfähig sind und somit unerwünschte Ablagerungen und Verstopfungen in Apparaten und Anlagen auftreten können.

Die Van-der-Waals Kräfte dieser kritischen Stoffe können durch Veränderung der Oberflächenmorphologie der Partikel beeinflusst werden. So lassen sich gemäss CA2492129 resp. der korrespondierenden WO 2004/007594 beispielsweise durch Aufbringen von Nanopartikeln an der Substratoberfläche die Adhäsionskräfte zwischen den Partikeln und den Partikeln und der Wand verringern (I. Zimmermann, M. Eber, K. Meyer, Nanomaterials as Flow Regulators in Dry Powders, Z. Phys. Chem. 218 (2004), 51-102). Trotz vereinzelter Studien in der Literatur (S. Jonat, S. Hasenzahl, A. Gray, P.C. Schmidt, Mechanism of Glidants: Investigation of the Effect of Different Collodial Silicon Dioxide Types on Powder Flow by Atomic Force and Scanning Electron Microscopy, J. Pharm. Sci. 93 (10) (2004), 2635-2644; J.H. Werth, M. Linsenbühler, S.M. Dammer, Z. Farkas, H. Hinrichsen, K.-E. Wirth, D.E. Wolf, Agglomeration of Charged Nanopowders in Suspensions, Powder Technol. 133 (2003), 106-112), die die Veränderung der Fliesseigenschaften von pharmazeutischen Stoffen durch Beigabe von Aerosil™ Nanopartikeln dokumentieren, steht bis dato kein marktgängiges bzw. -reifes Verfahren für die Verbesserung der Fliesseigenschaften von feinkörnigen Feststoffen zur Verfügung.

DARSTELLUNG DER ERFINDUNG

Die Erfindung dient demnach dazu, ein verbessertes Verfahren zur Anlagerung von Nanopartikeln an Substratpartikel zur Verfügung zu stellen. Insbesondere wird dabei erfindungsgemäss vorgeschlagen, die Bildung von Nanopartikeln und deren Anlagerung an Substratpartikeln mittels plasmaunterstützter chemischer Abscheidung aus der Gasphase zur Verfügung zu stellen.

Im folgenden wird definiert, wie die Begriffe "Nanopartikel" und "Substratpartikel" im Zusammenhang mit diesem Dokument verstanden werden:

Die Substratpartikel sind

- Feststoffpartikel, die mit dem in diesem Dokument beschriebenen Prozess behandelt werden. Es kann sich bei den Partikeln um organische oder anorganische Partikel handeln, die Partikel können aus einem einzigen Material (homogen) bestehen, es kann sich aber auch um Mischungen handeln (heterogen), oder um beispielsweise beschichtete Partikel. Möglich sind beispielsweise Polymerpartikel, Partikel auf Basis von organischen Molekülen wie z.B. Lactose oder anorganischen Stoffen etc..

- Partikel, die bezüglich der chemischen Zusammensetzung und physikalischen Eigenschaften keine Einschränkungen besitzen. Um auf jeden Fall der Behandlung im Plasma oder unmittelbar hinter dem Plasma bei Afterglow standhalten zu können, sollten Sie bevorzugtermassen bis 70°C stabil sein, das heisst die Umwandlungstemperatur in eine andere chemische Form sollte wenigstens bei Raumtemperatur liegen, vorzugsweise wenigstens 50°C, bevorzugtermassen wenigstens 70°C betragen. Vorzugsweise sind die Partikel elektrisch nicht leitend.

- Partikel, deren charalcteristische Grösse typischerweise im Bereich von wenigen Mikrometern bis Millimetern liegt, vorzugsweise aber sogar im Bereich von einigen hundert Nanometern (500 nm) bis einigen hundert Mikrometern (500 $\mu$m). Die Partikel können unterschiedliche Form aufweisen. So beispielsweise können sie die Form von Plättchen, Kugeln, Stäbchen, Flocken haben, oder es kann sich um unregelmässige Bruchstücke handeln (zum Beispiel als Resultat eines Mahlprozesses). Typischerweise verfügen sie über einen mittleren Durchmesser ($d_{50}$) im Bereich von 1 $\mu$m 1 mm, bevorzugt sogar von 500 nm - 500 $\mu$m.

Die Nanopartikel sind

- Feststoffpartikel, die über eine chemische Reaktion aus dem Monomer entstehen, wobei es sich auch um eine Mischung von verschiedenen Monomeren handeln kann, respektive Monomere mit Additiven oder ähnlichem.

- Gebilde, die aus einem einzelnen oder aus mehreren Partikeln (Agglomerat) bestehen können.

- Partikel, deren charalcteristische Grösse typischerweise kleiner als 1 $\mu$m oder sogar kleiner als 500 nm ist. Die Nanopartikel besitzen entsprechend normalerweise einen mittleren Durchmesser ($d_{50}$) im Bereich von 0.5 nm- 500 nm. Sie können so klein sein, dass man mit einem Rasterelektronenmikroskop deren Anwesenheit nicht nachweisen kann. Bevorzugtermassen sind die Nanopartikel in ihrer mittleren Grösse in einem Bereich von 0.5 nm - 500 nm, insbesondere bevorzugt im Bereich von 1-10 nm. Die ideale Grösse der Nanopartikel hinsichtlich der Wirkung auf die Fliessfähigkeit der Substratpartikel bemisst sich unter anderem auch an der Grösse und der Form der Substratpartikel. Die Grösse der Nanopartikel ist über die Prozessparameter einstellbar.

Insbesondere wird vorgeschlagen, Nanopartikel mittels plasmagestützter chemischer Abscheidung aus der Gasphase zu bilden und sie an Substratpartikel anzulagern.

Konkret wird ein Verfahren zur Bildung von Nanopartikeln und deren Anlagerung an Substratpartikeln vorgeschlagen, welches dadurch gekennzeichnet ist, dass:

- ein Gasstrom durch eine Plasmazone geführt wird, in welcher eine elektrische Gasentladung zur Erzeugung eines anisothermen Plasmas, insbesondere zur Erzeugung von freien Ladungsträgern (LT) und angeregten neutralen Spezies (Angeregte), verwendet wird, wobei dem Gasstrom vor, in (Direkt-PECVD, direct Plasma Enhanced Chemical Vapour Deposition) oder nach (Remote-PECVD) der Plasmazone ein gasförmiges Monomer beigemischt wird, welches als Ausgangsmaterial für die chemische Reaktion zur Bildung der Nanopartikel dient, und wobei die freien Ladungsträger und angeregten neutralen Spezies direkt in der Plasmazone oder nach der Plasmazone dazu verwendet werden, das gasförmige Monomer in einen chemisch reaktiven Zustand und zu einer homogenen chemischen Reaktion zu bringen, so dass sich durch chemische Abscheidung aus der Gasphase Nanopartikel bilden, und

- dass die gebildeten Nanopartikel in einer Behandlungszone, durch welche ein Substratpartikel- und/oder Substratpartikel-Gas-Strom unter Einfluss der Gasströmung und/oder der Gewichtskraft geführt wird, an die Oberfläche der Substratpartikel durch die Kollision der beiden Partikelarten angelagert werden.

Dabei ist es gemäss einer ersten bevorzugten Ausführungsform möglich, die Anlagerung der Nanopartikel an die Substratpartikel unmittelbar in der Plasmazone stattfinden zu lassen. Dies ist möglich, indem in einem Gas-Substratpartikelstrom unter Einfluss der Gasströmung und der Gewichtskraft die Substratpartilcel durch eine Behandlungszone geführt werden, wobei der Gasstrom neben den Substratpartikeln ein gasförmiges Monomer enthält, welches als Ausgangsmaterial für die chemische Reaktion zur Bildung der Nanopartikel dient; indem in der Behandlungszone eine elektrische Gasentladung zur Erzeugung eines anisothermen Plasmas als Plasmazone verwendet wird, wobei die freien Elektronen resp. die LT und Angeregten dazu verwendet werden, das gasförmige Monomer in einen chemisch reaktiven Zustand und zu einer homogenen chemischen Reaktion zu bringen, so dass sich durch chemische Abscheidung aus der Gasphase Nanopartikel bilden; und indem die gebildeten Nanopartikel direkt innerhalb der Plasmazone an die Oberfläche der Substratpartikel durch die Kollision der beiden Partikelarten angelagert werden. Die Tatsache, dass ein Gas-Substratpartikelstrom durch die Behandlungszone geführt wird führt dazu, dass in der Behandlungszone sowohl die Nanopartikel gebildet werden sowie auch die Substratpartikel gewissermassen in situ mit diesen Partikeln beschichtet werden.

Plasmazone und Behandlungszone können somit räumlich zusammenfallen und somit die Bildung der Nanopartikel und ihre Anlagerung unmittelbar gleichzeitig stattfinden. Alternativ ist möglich, dass die Behandlungszone der Plasmazone im wesentlichen unmittelbar nachfolgt (hier als Afterglow-Verfahren bezeichnet), wobei sich in letzterem Fall bevorzugtermassen der Gasstrom aus der Plasmazone und der Substratpartikel-Strom gewissermassen kreuzen. Durch die Plasmazone wird also in diesem Fall nur ein Gasstrom ohne Substratpartikel geführt. Erst unmittelbar hinter der Plasmazone wird der Gasstrom, welcher nun nicht mehr das Monomer sondern die in der Plasmazone gebildeten Nanopartikel trägt, mit einem Gasstrom, welcher die Substratpartikel führt (Substratpartikel-Strom), gewissermassen gekreuzt. Dieses auch Vorgehen ist sogar für noch temperatursensiblere Substratpartikel geeignet.

Bevorzugtermassen liegt die mittlere Verweilzeit der Substratpartikel in der Behandlungszone zwischen 10 ms und 1s.

Hierzu ist es in einer bevorzugten Ausführungsform möglich, dass die Substratpartikel ein einziges Mal durch die Behandlungszone geführt werden. Dabei können die Substratpartikel gewissermassen durch die Behandlungszone fallen. Dies ist vorzugsweise in einem Fallrohrreaktor realisierbar. Andererseits ist es aber auch möglich, dass die Substratpartikel in dem Substratpartikel-Gasstrom durch die Behandlungszone nach oben steigen. Diese Art der Substratpartikelführung geschieht vorzugsweise in einem Steigrohrreaktor.

In einer weiteren bevorzugten Ausführungsform ist es aber auch möglich, dass die Substratpartikel mehrmals z.B. periodisch durch die Behandlungszone geführt werden. Wobei sich die Behandlungszone dann vorzugsweise im Steigrohr einer zirkulierenden Wirbelschicht befindet.

In einer weiteren Ausführungsform ist es aber ebenso möglich, dass die Substratpartikel in der Behandlungszone verweilen. Hierbei wäre die Behandlungszone vorzugsweise in einem Trommelreaktor oder in einem Wirbelschichtreaktor angesiedelt.

Die Substratpartikel und der Gasstrom können an unterschiedlichen Stellen im Reaktor eingespeist werden.

Grundsätzlich kann es sich beim Monomer um eine chemische Substanz handeln, welche unter Einfluss der in der Plasmazone erzeugten LT und der Angeregten polymerisiert oder zu einem Oxid, vorzugsweise zu Siliziumoxid (SiO$_x$) reagiert. Letzteres erscheint, da chemisch inert, besonders geeignet und kann beispielsweise mittels Hexamethyldisiloxan (HMDSO) oder Mischungen enthaltend diese Komponente erhalten werden. Es können aber auch andere Monomere verwendet werden, welche unter den angegebenen Bedingungen Nanopartikel bilden. In Frage kommen Alkane, Alken und Alkine, wie z.B. Ethin (Trivialname: Acetylen), aber auch Kohlenwasserstoffe mit funktionellen Gruppen. Einsetzbar sind darüber hinaus Fluorkohlenwasserstoffe wie z.B. C$_2$F$_6$ oder metall-organische Monomere wie z.B. bereits benanntes HMDSO, Tetraethoxysilan oder Titanium (IV)isopropoxid. Denkbar sind aber auch Silane oder Titaniumtetrachlorid, um nur einige zu nennen. Hiernach ist es möglich, dass sowohl gasförmige als auch flüssige Monomere verwendet werden, letztere insbesondere bevorzugt in Form eines Dampfes oder aber auch eines Aerosols. Bezüglich möglicher Monomersysteme sei verwiesen auf eine Zusammenstellung von Morosoff (N. Morosoff, An Introduction to Plasma Polymerization, in: Plasma Deposition, Treatment, and Etching of Polymers, Editor: R. d'Agostino, Academy Press, San Diego 1990), die darin genannten Systeme seien in die Offenbarung ausdrücklich mit eingeschlossen.

Die chemische Reaktion kann über mehrere Reaktionsstufen ablaufen, und die Nanopartikel können untereinander kollidieren und agglomerieren, bevor sie sich an der Substratoberfläche anlagern und/oder die Nanopartikel auf der Substratoberfläche mit anderen Nanopartikeln kollidieren und agglomerieren. Die freien, noch nicht angelagerten Nanopartikel können durch heterogene chemische Abscheidung aus der Gasphase beschichtet werden und/oder die bereits an der Substratoberfläche angelagerten Nanopartikel können durch heterogene chemische Abscheidung aus der Gasphase beschichtet werden. Die noch nicht oder nur unwesentlich durch Nanopartikel beladene Substratoberfläche kann auch durch heterogene chemische Abscheidung aus der Gasphase beschichtet werden, und/oder die Substratoberfläche kann ausschliesslich durch heterogene chemische Abscheidung aus der Gasphase beschichtet werden.

Gemäss einer weiteren bevorzugten Ausführungsform wird zur Erzeugung einer elektrischen Gasentladung eine Mikrowellenkopplung, Mittel- oder Hochfrequenzkopplung oder DC-Anregung verwendet. In der Plasmazone kann also bevorzugtermassen ein anisothermes Niederdruckplasma oder ein anisothermes Normaldruckplasma vorliegen. Das Niederdruckplasma wird bevorzugtermassen bei einem Druck im Bereich von 0.27 mbar bis 2.7 mbar betrieben.

Gemäss einer weiteren bevorzugten Ausführungsform wird das Monomer in einem Gasstrom, insbesondere bevorzugt in einem Inertgasstrom (zum Beispiel Ar), zugeführt, wobei der Anteil des Monomerpartialdrucks am Gesamtdruck am Punkt der Zugabe in das Reaktionsvolumen im Bereich von 1 - 10 % (insbesondere bei HMDSO) liegt, insbesondere bevorzugt im Bereich von 2 - 5 %.

Generell erscheint ein Verfahren zur Erhöhung der Fliessfähigkeit sinnvoll, wenn Substratpartikel mit einer mittleren Grösse im Bereich von 1 $\mu$m - 1 mm, oder besonders im Bereich von 500 nm - 500 $\mu$m, insbesondere bevorzugt im Bereich von 5 $\mu$m - 500 $\mu$m in den Prozess eingeführt werden, wobei die Substratpartikel vorzugsweise elektrisch nicht leitend sind. Bei grösseren Substratpartikeln erscheint eine Behandlung im Sinne der Erfindung i.d.R. ohne wesentliche Vorteile, da zwischen Substratpartikeln ab einer Grösse von 20 $\mu$m die Van der Waals-Kräfte zunehmend an Bedeutung gegenüber der Gewichtskraft verlieren. Bevorzugtermassen findet das Verfahren bei vergleichsweise temperatursensiblen Substratpartilceln Anwendung, so kann es sich bei den Substratpartikeln z.B. um Partikel handeln, welche bis zu einer Temperatur von wenigstens 70°C (typische Maximaltemperatur der schweren Teilchen in einem anisothermen Niederdruckplasma) stabil sind. Hinsichtlich der Nanopartikel erweist es sich als vorteilhaft, wenn diese eine mittlere Grösse im Bereich von 0.5 nm-1 $\mu$m oder von 0.5 nm - 500 nm aufweisen. Die Grösse der Nanopartikel lässt sich über die Prozessparameter einstellen, wobei beispielsweise zur Einstellung der Parameter die einfach feststellbare Fliessfähigkeit der behandelten Substratpartikel verwendet werden kann.

Das oben beschriebene Verfahren findet insbesondere bevorzugt Verwendung zur Erhöhung der Fliessfähigkeit von Substratpartikeln.

Des Weiteren betrifft die vorliegende Erfindung eine Vorrichtung zur Durchführung eines Verfahrens, wie es oben beschrieben wurde. Die Vorrichtung ist bevorzugtermassen dadurch gekennzeichnet, dass eine Plasmazone vorhanden ist, durch welche ein Gasstrom geführt wird, und in welcher eine elektrische Gasentladung zur Erzeugung eines anisothermen Plasmas, insbesondere zur Erzeugung von freien Ladungsträgern und angeregten neutralen Spezies, verwendet wird, wobei dem Gasstrom vor, in oder nach der Plasmazone ein gasförmiges Monomer beigemischt wird, welches als Ausgangsmaterial für die chemische Reaktion zur Bildung der Nanopartikel dient, und wobei die freien Ladungsträger und angeregten neutralen Spezies direkt in der Plasmazone oder nach der Plasmazone dazu verwendet werden, das gasförmige Monomer in einen chemisch reaktiven Zustand und zu einer homogenen chemischen Reaktion zu bringen, so dass sich durch chemische Abscheidung aus der Gasphase Nanopartikel bilden, und dass eine Behandlungszone vorhanden ist, durch welche ein Substratpartikel- und/oder Substratpartikel-Gas-Strom unter Einfluss der Gasströmung und/oder der Gewichtskraft geführt wird, und in welcher die gebildeten Nanopartikel an die Oberfläche der Substratpartikel durch die Kollision der beiden Partikelarten angelagert werden..

Eine erste bevorzugte Ausführungsform der Vorrichtung ist dadurch gekennzeichnet, dass ein erstes Führungselement, vorzugsweise in Form eines Rohres angeordnet ist, in welchem die Substratpartikel im Sinne eines Fallrohres oder eines Steigrohres geführt werden, und dass ein zweites, bevorzugtermassen im wesentlichen in einem rechten Winkel zum ersten Führungselement angeordnetes und in dieses erste Führungselement mündendes zweites Führungselement, vorzugsweise in Form eines Rohres, vorhanden ist, in welchem zweiten Führungselement der Gasstrom mit den Monomeren geführt ist und in welchem zweiten Führungselement die anisotherme Plasmazone angeordnet ist, sodass im wesentlichen unmittelbar hinter dieser Plasmazone die dort gebildeten Nanopartikel im Gasstrom an die Oberfläche der Substratpartikel durch die Kollision der beiden Partikelarten in der Behandlungszone angelagert werden.

Weitere bevorzugte Ausführungsformen sind in den abhängigen Ansprüchen beschrieben.

Die Behandlung von schlecht oder nicht fliessfähigen feinkörnigen Stoffen in anisothermen Plasmen mithilfe der chemischen Abscheidung von Nanopartikeln aus der Gasphase und dessen Anlagerung an die Substratpartikeloberfläche ist eine viel versprechende und für diese Anwendungen bisher nie in Betracht gezogenen Methode zur Verbesserung der Fliesseigenschaften.

Sogenannte anisotherme Gasentladungen sind dadurch charakterisiert, dass die Elektronen und die schweren Spezies nicht im thermodynamischen Gleichgewicht sind (A. Grill, Cold Plasma in Materials Fabrication, From Fundamentals to Applications, IEEE Press, Piscataway (1994)). Die Energie des Systems ist nicht gleichmässig auf alle Teilchenarten verteilt, sondern konzentriert sich hauptsächlich auf die kinetische Energie der Elektronen, deren Temperatur in der Grössenordnung von $10^4$ oder sogar $10^5$ K liegt. Da sich die mittlere Temperatur des gesamten Systems aus der mittleren kinetischen Energie aller Teilchen ergibt und die Temperatur der schweren Spezies 300-500 K beträgt, ist diese Art von Plasma besonders für die Behandlung von temperatursensiblen Stoffen, wie zum Beispiel pharmazeutischen Produkten, geeignet. Trotz niedriger Systemtemperatur genügt die kinetische Energie der Elektronen im unelastischen Stoss, die Aktivierungsenergie für eine chemische Reaktion zur Verfügung zu stellen.

Die Bildung von Nanopartikeln im Plasma wurde bereits in verschiedenen Patenten (US2005/118094, JP2004024953) und Arbeiten beschrieben. Vor allem die Generierung von Partikeln in thermischen Plasmen ist seit längerem bekannt (R.M. Young, E. Pfender, Generation and Behavior of Fine Particles in Thermal Plasmas - a Review, Plasma Chem. Plasma Process. 5 (1) (1985), 1-37; N. Rao, S. Girshick, J. Heberlein, P. McMurry, S. Jones, D. Hansen, B. Micheel, Nanoparticle Formation Using a Plasma Expansion Process, Plasma Chem. Plasma Process. 15 (4) (1995), 581-606) und wird auch in verschiedensten Industriezweigen, wie zum Beispiel der Pulvermetallurgie, angewandt. Die hohen Temperaturen von 1000 K und mehr führen zwar zu hohen Umsatzraten, sind jedoch im Zusammenhang mit der Behandlung von thermisch instabilen Materialien ungeeignet.

Über die Nanopartikelbildung in anisothermen Plasmen, welche Bestandteil der vorliegenden Patentschrift ist, gibt es ebenfalls verschiedenste Literaturangaben.

Bei den bekannten Oberflächenbehandlungsprozessen, welche eine homogene Beschichtung des Substrates voraussetzen, wird die Partikelbildung im Plasma stets als störender Nachteil angesehen (G.S. Selwyn, J. Singh, R.S. Bennett, In situ laser diagnostic studies of plasma-generated particulate contamination, J. Vac. Sci. Technol. A 7 (4) (1989), 2758-2765), da durch die Partikelkontaminierung unerwünschte Materialdefekte auftreten können. Heutzutage jedoch wird an sich die Nanopartikelbildung in elektrischen Entladungen nicht mehr ausschliesslich als ungewollte Verschmutzung betrachtet. So können die kleine Partikelgrösse (Nanometerbereich), die uniforme Partikelgrössenverteilung, oder die chemische Aktivität der im Plasma gebildeten Partikel, als nützliche Eigenschaften angesehen werden (H. Kersten, G. Thieme, M. Fröhlich, D. Bojic, D.H. Tung, M. Quaas, H. Wulff, R. Hippler, Complex (dusty) plasmas: Examples for applications and observation of magnetron-induced phenomena, Pure Appl. Chem. 77 (2) (2005), 415-428). Die Partikelbildung wurde sowohl für Hochfrequenz-, wie auch für Mikrowellen-Kopplungen untersucht, wobei Prozessparameter wie Systemdruck, eingetragene Leistung, Temperatur, Verweilzeit und Monomerkonzentration variiert wurden (T. Fujimoto, K. Okuyama, M. Shimada, Y. Fujishige, M. Adachi, I. Matsui, Particle generation and thin film surface morphology in the tetraethylorthosilicate/oxygen plasma enhanced chemical vapor deposition process, J. Appl. Phys. 99 (5) (2000), 3047-3052; A. Bouchoule, A. Plain, L. Boufendi, J.Ph. Blondeau, C. Laure, Particle generation and behavior in silane-argon low-pressure discharge under continuous or pulsed-frequency excitation, J. Appl. Phys. 70 (4) (1991), 1991-2000; J.H. Chu, L. I, Fine silicon oxide particles in rf hollow magnetron discharges, J. Appl. Phys. 74 (7) (1993), 4741-4745; S. Schlabach, V. Szabo, D. Vollath, A. Braun, R. Clasen, Structure of Alumina and Zirconia Nanoparticles Synthesized by the Karlsruhe Microwave Plasma Process, Solid State Phenomena, 99-100 (2004), 191-196).

Mögliche apparative Anordnungen und Reaktortypen für die Plasmabehandlung von Feststoffpartikeln sind u.a. in folgenden Patentdokumenten beschrieben: EP 0 807 461, US 5,620,743, US 4,685,419, US 5,234,723 und US 2004/182293. Am wenigsten verbreitet sind Batchreaktoren mit Rührorgan (J.W. Kim, Y.S. Kim, H.S. Choi, Thermal characteristics of surface-crosslinked high density polyethylene beads as a thermal energy storage material, Korean J. Chem. Eng.19 (4) (2003), 632-637), die nur eine unregelmässige Behandlung der Partikel zulassen, da die Partikel in diesen Reaktoren einen Körper bilden, welcher nur an der Oberfläche mit dem Plasma in Kontakt kommt.

Ähnliches gilt für Trommelreaktoren (US 5,925,325), welche eine hohe Durchsatzleistung erlauben.

Die zirkulierende Wirbelschicht (M. Karches, Ch. Bayer, Ph. Rudolf von Rohr, A circulating fluidised bed for plasma-enhanced chemical vapor deposition on powders at low temperatures, Surf. Coat. Tech. 119 (1999), 879-885) bietet gegenüber der herkömmlichen Wirbelschicht (Ch. Bayer, M. Karches, A. Matthews, Ph. Rudolf von Rohr, Plasma Enhanced Chemical Vapor Deposition on Powders in a Low Temperature Plasma Fluidized Bed, Chem. Eng. Technol. 21 (5) (1998), 427-430) die Vorteile, dass eine homogenere Partikelbehandlung und eine engere Verweilzeitverteilung erreichbar sind.

Der kontinuierlich geführte Fallrohrreaktor (C. Arpagaus, A. Sonnenfeld, Ph. Rudolf von Rohr, A Downer Reactor for Short-time Plasma Surface Modification of Polymer Powders, Chem. Eng. Technol. 28 (1) (2005), 87-94) bietet die Möglichkeit einer homogenen Kurzzeitbehandlung der Partikel.

Die Idee der Erfindung liegt darin, dass durch ein nichtthermisches (d.h. anisothermes) Plasma Nanopartikel aus der Gasphase abgeschieden und im selben Prozessschritt an die Substratpartikel angelagert werden, um so beispielsweise die Fliesseigenschaften von feinkörnigen Stoffen zu verbessern. Dadurch, dass die schweren Spezies im Plasma nicht im thermodynamischen Gleichgewicht mit den Elektronen sind, ist es insbesondere möglich, temperatursensible Stoffe zu behandeln.

Partikeladhäsionseffekte, welche zum Beispiel durch Elektrostatik, Flüssigkeitsbrücken oder Van-der-Waals Kräfte verursacht werden, führen dazu, dass die Fliessfähigkeit von Feststoffen stark herabgesetzt wird. Bei Partikelgrössen kleiner 20 $\mu$m dominiert die Van-der-Waals Wechselwirkung gegenüber allen anderen Kräften. Neben dem Partikelradius hängt sie vor allem vom Abstand der Substratpartikeloberflächen ab. Mit zunehmendem Partikelradius und abnehmendem Partikelabstand wächst die Van-der-Waals stark an. Eine Möglichkeit, dieses Problem zu umgehen, ist das Aufbringen von noch kleineren Partikeln mit Durchmessern im Nanometerbereich auf der Partikeloberfläche, um so den Abstand zwischen den Substratpartikeln zu vergrössern und somit die Anziehungskräfte zu verkleinern.

Die Erfindung bezieht sich auf den Prozess, welcher sich in die Teilprozesse Partikelbildung und Anlagerung unterteilen lässt. Diese beiden Schritte sollen in den nächsten Abschnitten näher erläutert werden.

Die Bildung und das Wachstum der Nanopartikel im Plasma lässt sich in vier Phasen unterteilen (A. Bouchoule, Dusty Plasmas, Physics, Chemistry and Technological Impacts in Plasma Processing, Wiley, Chapter 2 (1999)). In einem ersten Schritt bilden sich Primärcluster aus den Atomen und/oder Molekülen des Precursorgases, welches sich über eine oder mehrere chemische Reaktionen aus dem Monomer gebildet hat. Während des Clusterwachstums bilden sich dann erste Partikelkeime, welche zu nanometergrossen Strukturen (< 5nm) anwachsen. In der dritten Phase kommt es zur Agglomeration der Primärpartikel, wobei die Formationen bis zu 50 nm gross werden können. Danach wachsen die Partikel durch Ablagerung aus der Gasphase unabhängig voneinander weiter.

Im zweiten Schritt des Prozesses kollidieren die im ersten Schritt gebildeten Nanopartikel mit den Substratpartikeln und bleiben auf Grund der Adhäsionskräfte an der Substratoberfläche haften. Dies setzt einen intensiven Kontakt der beiden Partikelspezies voraus, welcher sich zum Beispiel in einer Wirbelschicht realisieren lässt.

Die beiden Hauptzielgrössen des Produktes sind der Nanopartikeldurchmesser $d_{Np}$ und die Zahl der Nanopartikel $n_{NP}$ pro Substratpartikeloberfläche $A_{SP}$

$$\frac{n_{NP}}{A_{SP}} = \frac{2}{\sqrt{3}d_{NP}^2},$$

welche einen massgebenden Einfluss auf die Fliesseigenschaften der Substratpartikel haben. Grundsätzlich gilt die Regel, dass der Durchmesser der Nanopartikel so gewählt werden sollte, dass sich die Substratpartikel nicht direkt berühren können sondern stets nur über Nanopartikel. Die Nanopartikel dienen also dazu, die Kontaktfläche zu reduzieren und so die ungewünschte Haftung zwischen den Substratpartilceln weitgehend zu reduzieren. Die ideale Belegung der Oberfläche hängt unter anderem von der Form der Substratpartikel und deren Grösse ab, sowie von der Grösse und Form der Nanopartikel. Entsprechend ist die Anzahl der Nanopartikel pro Substratoberfläche gewissermassen indirekt über die gewünschte und leicht messbare Fliessfähigkeit einstellbar. Diese Zielgrössen können über die Prozessparameter, wie zum Beispiel Druck, Verweilzeit, Gaszusammensetzung, Temperatur oder Leistungseintrag im Plasma für die Gasentladung gesteuert werden.

Den in der Regel wichtigsten Parameter stellt der Systemdruck dar, da dieser einen wesentlichen Einfluss auf die Partikelbildung in anisothermen Plasmen hat. Der Systemdruck bzw. Reaktordruck ist der Druck im Plasma ; also vorzugsweise der Druck in der Plasmazone. Typischerweise kommt für das vorgeschlagene Verfahren ein Niederdruckplasma mit Drücken im Bereich von 0.27 - 2.7 mbar zur Anwendung. Im angegebenen Druckbereich werden bevorzugt Nanopartikel gebildet, da die mittlere Diffusionslänge (zur Gewährleistung homogener chemischer Reaktionen) bereits genügend klein ist, während die anfänglich nötige Fragmentierung bzw. Aktivierung des Monomers (bevorzugt ausgelöst durch hochenergetische Elektronen) aufgrund einer ausreichenden Elektronendichte, noch genügend gross ist (vgl. dazu generell z.B. N. Morosoff, An Introduction to Plasma Polymerization, in: Plasma Deposition, Treatment, and Etching of Polymers, Editor: R. d'Agostino, Academy Press, San Diego, 1990). So haben Versuche gezeigt, dass bei tiefen Drücken eine heterogene Gasphasenreaktion an der Oberfläche der Substratpartikel stattfindet und dort somit eine homogene Schichtenbildung gefördert wird. Bei höheren Prozessdrücken jedoch wird die Frequenz der Teilchenkollisionen im Plasma erhöht, so dass die homogene Gasphasenreaktion bevorzugt stattfindet und somit die Partikelbildung begünstigt wird.

Die Verweilzeit der zu behandelnden Substratpartikel im Reaktor, oder genauer in der Behandlungszone, sowie des Gasstromes in der Plasmazone stellen ebenfalls wichtige Grössen des Prozesses dar. So lassen sich über diese Parameter die Grösse der Nanopartikel und die Anzahl Nanopartikel pro Substratoberfläche steuern. Versuche zeigen, dass eine Behandlungszeit in der Grössenordnung von einer Zehntelsekunde genügt, um das erwünschte Resultat zu erzielen. Es ist aber auch möglich, die Substratpartikel mehrfach durch die Behandlungszone zu zirkulieren. Je länger die Verweilzeiten in Plasmazone und Behandlungszone sind, um so grössere Nanopartikel bilden sich und respektive um so besser wird die Belegung der Oberfläche. Generell kann gesagt werden, dass eine gesamte Verweilzeit in der Behandlungszone und/oder in der Plasmazone (gegebenenfalls auch bei zyklischer Exposition der Substratpartikel als Summe, d.h. akkumuliert, zu verstehen) im Bereich von 10 ms - 1 s geeignet ist. Folglich muss das Reaktorkonzept so gewählt werden, dass solch kurze Verweilzeiten mit entsprechend enger Verweilzeitverteilung der Substratpartikel eingehalten werden können. Dafür geeignete Konfigurationen sind zum Beispiel der eingangs genannte Fallrohrreaktor oder die eingangs genannte zirkulierende Wirbelschicht, welche beide weiter unten im Detail beschrieben werden sollen .

Der von uns beschriebene Prozess bietet verschiedene Vorteile:

- Die Fliessfähigkeit von feinkörnigen Stoffen, bei welchen die Van-der-Waals Kräfte gegenüber der Gravitation und den anderen Partikelinteraktionskräften dominieren, lässt sich durch das geschilderte Verfahren verbessern. Dadurch lassen sich Verstopfungen und Ablagerungen in Apparaten bei Vorgängen wie Mischen, Austragen oder Dosieren vermeiden, was wiederum mit Zeit- und Kosteneinsparungen verbunden ist.

- Die beiden Teilschritte Nanopartikelbildung und Anlagerung fallen in einem Prozessschritt zusammen oder werden unmittelbar nacheinander im Gasstrom durchgeführt. Eine zusätzliche Verarbeitung von Nanopartikeln, welche wiederum mit Adhäsionserscheinungen und gesundheitsgefährdenden Aspekten verbunden ist, entfällt somit. Zudem ist die Gefahr einer Staubexplosion im Niederdruck vernachlässigbar klein.

- Im Vergleich zu Verfahren, wo die Nanopartikel über einen Mischprozess an die Substratoberfläche angelagert werden (vergleiche beispielsweise I. Zimmermann, M. Eber, K. Meyer, Nanomaterials as Flow Regulators in Dry Powders, Z. Phys. Chem. 218 (2004), 51-102), ergeben sich deutlich kürzere Behandlungszeiten (Stunden gegenüber Sekunden oder Bruchteilen von Sekunden) und damit eine wesentliche Verbesserung der Wirtschaftlichkeit.

- Die tiefen Prozesstemperaturen (<70°C) ermöglichen es, temperaturempfindliche Substratpartikel zu behandeln. Wie bereits erläutert handelt es sich um ein Niederdruckplasma, bei welchem im wesentlichen nur die Elektronen eine hohe kinetische Energie besitzen (Temperaturäquivalent im Bereich von 1000-10'000 K). Entsprechend werden in der Regel weder Substratpartikel noch Monomer noch Nanopartikel im Prozess auf eine Temperatur oberhalb von circa 70°C erwärmt.

Zusammenfassend lässt sich das bevorzugte Verfahren wie folgt charakterisieren:

Eine Apparatur und ein Verfahren zur Bildung von Nanopartikeln und deren Anlagerung an Substratpartikel, das durch folgende Merkmale gekennzeichnet ist: - Die Verwendung eines Gas-Substratpartikelstroms, der unter Einfluss der Gasströmung und der Gewichtskraft die Substratpartikel durch die so genannte Behandlungszone führt (z.B. Fall- oder Steigrohr); - Die Verwendung eines Gasstromes, der neben anderen Spezies das gasförmige Monomer enthält, welches als Ausgangsmaterial für die chemische Reaktion benutzt wird; - Die Verwendung einer elektrischen Gasentladung zur Erzeugung eines anisothermen Plasmas, in dem die freien Elektronen (genauer: LT und Angeregten, vorzugsweise hochenergetische Elektronen) dazu verwendet werden, das gasförmige Monomer in einen chemisch reaktiven Zustand zu bringen; - Der Vorgang der homogenen chemischen Reaktion der reaktiven Spezies in der Gasphase; - Der Vorgang der Bildung von Nanopartikeln, welche durch die chemische Abscheidung aus der Gasphase hervorgerufen wird; - Der Vorgang der Anlagerung der Nanopartikel an die Oberfläche der Substratpartikel, welcher durch die Kollision der beiden Partikelarten hervorgerufen wird und direkt innerhalb der Plasmazone stattfindet.

Eine Apparatur und ein Verfahren dieser Art kann dadurch gekennzeichnet sein, dass der Vorgang der Ablagerung der Nanopartikel an die Oberfläche der Substratpartikel ausserhalb der Plasmazone stattfindet. Es kann auch dadurch gekennzeichnet sein, dass anstelle der Verwendung eines eigen für das Verfahren konzipierten Reaktors der beschriebene Prozess in einem anderen Verfahrensschritt bzw. in einer anderen Apparatur (z.B. Strahlmühle) integriert wird. Es kann weiterhin dadurch gekennzeichnet sein, dass anstelle des in der detaillierten Beschreibung einer möglichen technischen Umsetzung der Erfindung beschriebenen $SiO_x$ ein anderes Reaktionsprodukt entsteht, welches die Grundlage für die Nanopartikelerzeugung bildet.

KURZE ERLÄUTERUNG DER FIGUREN

Die Erfindung soll nachfolgend anhand von Ausführungsbeispielen im Zusammenhang mit den Zeichnungen näher erläutert werden. Es zeigen:

Figur 1     eine zirkulierende Wirbelschicht als mögliche Anlage zur Realisierung des Prozesses; der Vorgang, welcher in in diesem Dokument beschrieben wird, findet hauptsächlich innerhalb der aktiven Plasmazone des Reaktors statt; alle wesentlichen, die Gesamtanlage betreffenden Bauteile sind für die Beschreibung des Aufbaus jeweils nummeriert;

Figur 2     einen Fallrohrreaktor als mögliche Anlage zur Realisierung des Prozesses;

Figur 3     Fliessfähigkeiten der Substratpartikel;

Figur 4     Fliessfähigkeit in Abhängigkeit der Monomerflussrate; und

Figur 5     Fliessfähigkeit in Abhängigkeit der RF-Lesitung im Plasma..

WEGE ZUR AUSFÜHRUNG DER ERFINDUNG

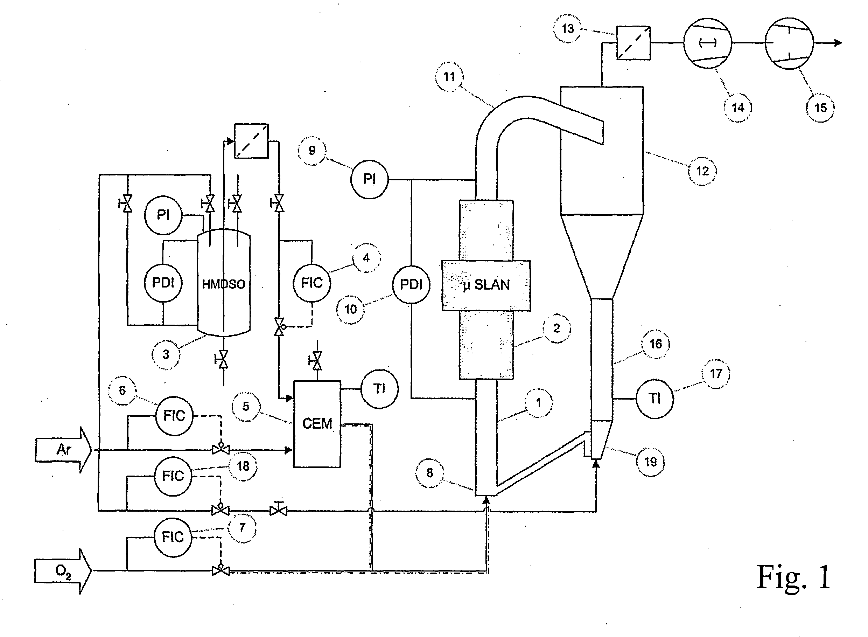

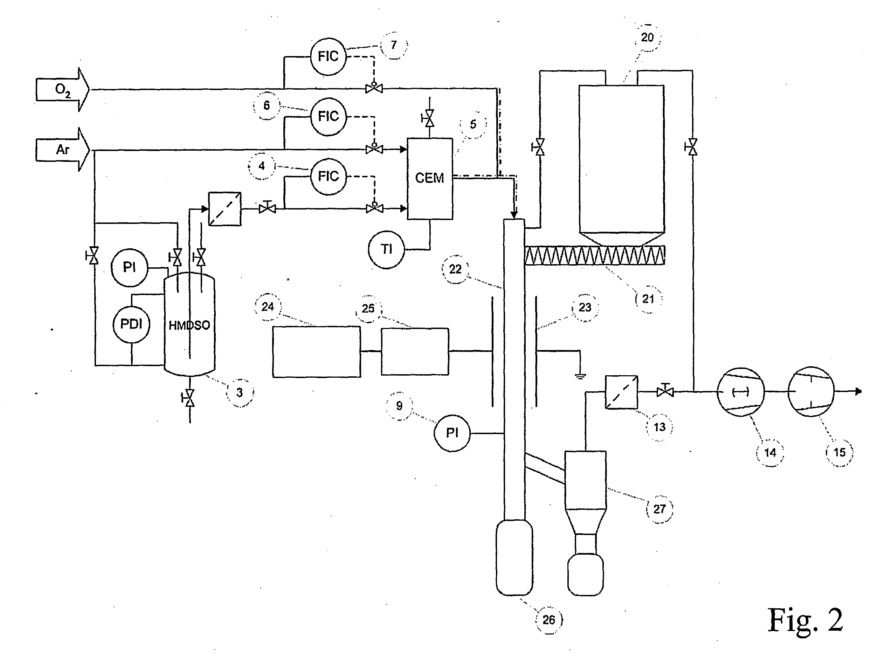

Anhand der zirkulierenden Wirbelschicht (Figur 1) und dem Fallrohrreaktor (Figur 2) soll beispielhaft gezeigt werden, wie die Erfindung realisiert werden kann. Als Monomer wird HMDSO verwendet, welches unter geeigneten Bedingungen zu $SiO_x$ reagiert und schliesslich in Form von Nanopartikeln an die Oberfläche der zu behandelnden Substratpartikel angelagert wird.

Zirkulierende Wirbelschicht:

Kernstück der Anlage ist das Steigrohr 1 aus Quarzglas, in welchem die zu behandelnden Substratpartikel durch die Plasmazone geführt werden. Mit Hilfe der Mikrowellenplasmaquelle 2 μSLAN (JE Plasma Consult, Deutschland) werden die Mikrowellen über ein Ringresonator-Schlitzantennenprinzip in die Plasmazone abgestrahlt, wodurch sich das Plasma innerhalb des Quarzrohres ausbilden kann. Die Mikrowellenanregung findet bei einer Frequenz von 2.45 GHz statt, wobei die Vorwärtsleistung zwischen 0 und 2000 W variierbar ist. Generell kann beobachtet werden, dass eine höhere Leistungseinkopplung auch zu einem besseren Fliessverhalten der Substratpartikel führt.

Das Prozessgas setzt sich aus Argon, Sauerstoff und HMDSO zusammen. Letzteres wird in einem Druckbehälter 3 in flüssiger Form gespeichert und über einen Massendurchflussregler 4 zum Verdampfungsmodul 5 geführt, wo die Verdampfung des Monomers erfolgt. Generell gilt, dass ein um so besseres Fliessverhalten der bearbeiteten Substratpartikel erreicht werden kann, je mehr Monomer zugeführt werden kann (normalerweise circa eine Anteil des Monomerpartialdrucks am Gesamtdruck, d.h. am Systemdruck von 2% bis 10 %). Gleichzeitig wird an dieser Stelle im Verdampfungsmodul Argon zugemischt. Seine Flussmenge wird ebenfalls über einen Massendurchflussregler 6 eingestellt. Um eine erneute Kondensation des Monomers zu verhindern, wird das Gasgemisch bis zum Reaktoreintritt beheizt. Zusätzlich erfolgt die Beimischung des Sauerstoffs über einen weiteren Massendurchflussregler 7. Schliesslich strömt das Prozessgas über eine Sinterplatte 8 (diese Platte lässt nur Gas hindurch und hält Substratpartikel zurück) in den Reaktor und führt somit zu einer Dispergierung des Feststoffes. Durch die Reibungskräfte des Gases werden die Substratpartikel in vertikaler Richtung beschleunigt. Die Substratpartikel werden bei diesem Reaktor vor dem Start eines Batches vorgelegt und anschliessend in dem mit den Bezugszeichen 1, 2, 11, 12, 16, 19 bezeichneten Kreislauf zirkuliert. Es handelt sich mit anderen Worten bei diesem Reaktor um einen Reaktor, bei welchem die Substratpartikel mehrfach durch die Behandlungszone geführt werden, und es handelt sich um einen Prozess, welcher normalerweise nicht kontinuierlich geführt werden kann.

Am Ende des Steigrohrs wird der Systemdruck resp. Prozessdruck mit einer kapazitiven Drucksonde 9 gemessen. Mittels Bestimmung des Druckabfalls 10 über die Plasmazone lässt sich der Feststoffmassenstrom im Rohr abschätzen.

Nach der Plasmazone gelangt der Gas-Partikelstrom über den gekrümmten Einlauf 11 in den Zyklonabscheider 12. Das Gas wird durch einen Staubfilter 13 von den Vakuumpumpen abgeführt. Das zweistufige Pumpensystem besteht aus einer Wälzkolbenpumpe 14 und einer zweistufigen Drehschieberpumpe 15.

Die im Zyklon abgeschiedenen Partikel werden im Fallrohr 16 gespeichert. In diesem Bereich des Reaktors wird ebenfalls die Temperatur der Substratpartikel mittels Thermoelement 17 gemessen. Ein zusätzlicher Argonstrom, welcher über einen Massendurchflussregler 18 gesteuert wird, fluidisiert das Festbett im untersten Bereich des Fallrohres 19 und ermöglicht dadurch eine gleichmässige Rückführung der Substratpartikel in das Steigrohr. Über den Argonstrom lässt sich der Fluidisierungsgrad der Schüttung und somit auch der Massenstrom der in das Steigrohr zurückgeführte Partikel steuern.

Fallrohrreaktor:

Die Prozessgaszuführung 3-7 sowie das Pumpensystem 13-15 sind identisch mit denjenigen der zirkulierenden Wirbelschicht und werden an dieser Stelle nicht näher erläutert.

Im Gegensatz zur zirkulierenden Wirbelschicht kann der Fallrohrreaktor kontinuierlich betrieben werden. Die unbehandelten Substratpartikel werden in einem Vorratsbehälter 20 gespeichert, bevor sie über eine drehzahlregelbare Förderschnecke 21 in das Fallrohr 22 aus Quarzglas transportiert werden.

Das von oben in das Fallrohr einströmende Prozessgas beschleunigt die Substratpartikel in vertikaler Richtung nach unten. Damit eine homogene Partikelbehandlung erreicht werden kann, wird der Feststoff über eine Düse (vgl. diesbezüglich z.B. C. Arpagaus, A. Sonnenfeld, Ph. Rudolf von Rohr, A Downer Reactor for Short-time Plasma Surface Modification of Polymer Powders, Chem. Eng. Technol. 28 (1) (2005), 87-94) homogen über den Rohrquerschnitt dispergiert. Anschliessend strömt das Gas-Feststoffgemisch durch die Reaktionszone, wo das Plasma über zwei kapazitiv gekoppelte Elektroden 23 (normalerweise Kupferelektroden) erzeugt wird. Im Gegensatz zur Mikrowelle wird hier die Energie mit einem Hochfrequenzgenerator (13.56 MHz, PFG 300, Hüttinger Electronic, Deutschland) erzeugt, wobei die Vorwärtsleistung zwischen 0 und 300 W eingestellt werden kann. Das Anpassungsnetzwerk 25 (PFM 1500A, Hüttinger Electronic, Deutschland) zwischen Generator 24 und gespeister Elektrode sorgt für die Impedanzanpassung.

Unterhalb der Plasmazone wird der Prozessdruck mittels kapazitiver Drucksonde 9 gemessen. Der grösste Teil der behandelten Substratpartikel wird in einem Auffangbehälter 26 gesammelt. Der restliche Feststoff wird über den Zyklon 27 abgeschieden.

Entsprechend findet beim Fallrohrreaktor keine zyklische Behandlung der Substratpartikel statt, die Substratpartikel fallen vielmehr ein einziges Mal durch das Fallrohr zur Beschichtung mit Nanopartikeln. Der Prozess ist aber im Gegensatz zur zirkulierenden Wirbelschicht ein kontinuierlicher Prozess.

Anwendungsbeispiel 1:

Anhand einer Modellsubstanz ($\alpha$-D-Lactose Monohydrat, $d_{50}$ = 5.5 $\mu$m) soll gezeigt werden, wie die Fliesseigenschaften von Feststoffen mit Hilfe des vorgestellten Prozesses erheblich verbessert werden können.

Die Behandlung der Partikel wurde im oben beschriebenen Fallrohrreaktor durchgeführt.

Der Ablauf des Behandlungsprozesses lässt sich folgendermassen beschreiben:

- Nachdem der Vorratsbehälter mit den unbehandelten Laktosepartikeln gefüllt wurde, werden der Reaktor vakuumdicht geschlossen und die Vakuumpumpen eingeschaltet. Der Rezipient wird bis zu einem Absolutdruck von 0.05 mbar evakuiert.

- Die Massendurchflussregler werden so eingestellt, dass 50 sccm Argon und 1030 sccm (standard cubic centimeters per minute) Sauerstoff in den Reaktor strömen. Es wird ein Prozessdruck von 2 mbar eingestellt, welcher für den weiteren Verlauf des Versuchs konstant gehalten wird.

- Der RF-Generator (Vorwärtsleistung 100 W) wird eingeschaltet und die Kapazitäten des Anpassungsnetzwerkes werden so eingestellt, dass die reflektierte Leistung < 10 W beträgt. Damit wird eine effektive Leistung von > 90 W erzeugt. Das Plasma zündet.

- Der Massendurchflussregler für das Monomer wird so eingestellt, dass dem Prozessgasstrom 103 sccm HMDSO ($\geq$ 98.5 %, Fluka) beigemischt werden.

- Nachdem sich ein stationärer Zustand im Reaktor eingestellt hat, kann die Förderschnecke für die Feststoffzufuhr eingeschaltet werden, so dass kontinuierlich 1.3 kg Laktose pro Stunde durch den Reaktor geführt werden (Verweilzeit $\approx$ 0.1 s).

- 180 s nach dem Einschalten der Förderschnecke wird diese wieder ausgeschaltet. Es wird kein Feststoff mehr gefördert.

- Die Zufuhr des Monomers wird unterbrochen und der RF-Generator ausgeschaltet. Das Plasma erlischt.

- Die restliche Prozessgaszufuhr (Argon, Sauerstoff) wird unterbrochen und das Ventil zwischen Pumpe und Rezipient geschlossen.

- Der Reaktor kann nun auf Atmosphärendruck gebracht und die behandelte Laktose aus dem Auffangbehälter entnommen werden.

Resultate:

Die Fliessfähigkeit ($ff_c$, zur Definition vgl. D. Schulze, Zur Fliessfähigkeit von Schüttgütern - Definition und Messverfahren, Chem. Ing. Tech. 67 (1) (1995), 60-68) der behandelten und unbehandelten Laktose wird mit Hilfe eines Ringschergerätes (RST-XS, Schulze Schüttgutmesstechnik, Deutschland, vgl. auch D. Schulze, A. Wittmaier, Flow Properties of Highly Dispersed Powders at Very Small Consolidation Stresses, Chem. Eng. Technol. 26 (2) (2003), 133-137) gemessen. Die dazu verwendete Scherzelle hat ein Fassungsvermögen von 30 ml. Die bei der Messprozedur angewandte Anscherspannung beträgt 5000 Pa, wobei Abscherspannungen 1000, 2500 und 4000 Pa gewählt werden.

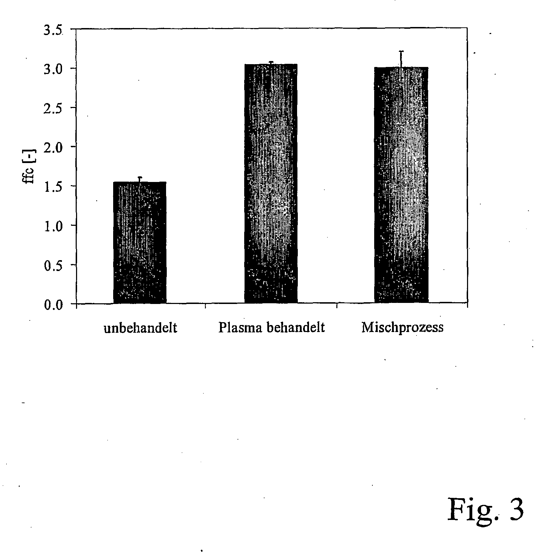

Die gemessenen Fliessfähigkeiten sind Abbildung 3 zu entnehmen. Die Fehlerbalken beziehen sich auf ein 95%-Vertrauensintervall. Es ist erkennbar, dass sich die Fliessfähigkeit der unbehandelten Laktose durch den beschriebenen Plasmaprozess von 1.5 (sehr kohäsiv) auf 3 (kohäsiv) verbessern lässt. Als Vergleich dazu ist die Fliessfähigkeit von Laktosepartikeln dargestellt, welche mit Aerosil™ in einem konventionellen Mischprozess (Mischzeit: 8 h) behandelt wurden (vgl. z.B. P. Reichen, Tailoring Particle Properties of Fine Powders by Surface Modifiers, Private Commun., Diploma Thesis, ETH Zürich 2005).

Anwendungsbeispiel 2:

Anhand einer Parameterstudie wird gezeigt, inwiefern sich die Monomerflussrate auf die Fliesseigenschaften der Modellsubstanz ($\alpha$-D-Lactose Monohydrat, $d_{50}$ = 5.5 $\mu$m) auswirkt. Der Aufbau des Reaktors, sowie der Ablauf des Behandlungsprozesses sind identisch mit denjenigen aus dem Anwendungsbeispiel 1, mit der Ausnahme, dass die Prozessgaszusammensetzung variiert wird.

- Um ein konstantes Sauerstoff zu HMDSO-Verhältnis von 10 gewährleisten zu können, wird entsprechend die Sauerstoffflussrate (170 - 1030 sccm) der Monomerflussrate (17 -103 sccm) angepasst.

- Damit die Verweilzeit in der Plasmazone für alle Parametereinstellungen konstant bleibt, muss die Geschwindigkeit des Prozessgases im Fallrohr konstant gehalten werden. Dementsprechend wird das Sauerstoff-HMDSO-Gemisch mit Argon (50 - 995 sccm) ergänzt, so dass sich ein konstanter Gasfluss von 1083 sccm einstellt. Bei einem Prozessdruck von 2 mbar entspricht dies einer Gasgeschwindigkeit von ungefähr 8 m/s.

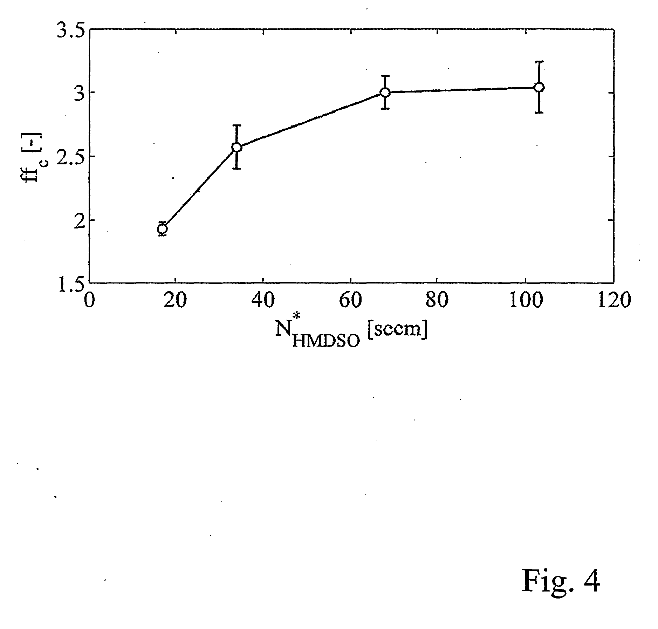

Die Fliessfähigkeiten der behandelten Pulver werden mit dem im Anwendungsbeispiel 1 beschriebenen Verfahren gemessen. Aus Abbildung 4 ist erkennbar, dass die Fliessfähigkeit mit zunehmender HMDSO-Flussrate zunimmt, was auf eine erhöhte Abscheidungsrate bei zunehmendem Partialdruck des Monomers zurückzuführen ist.

Anwendungsbeispiel 3:

Anhand einer weiteren Parameterstudie wird gezeigt, inwiefern sich die RF-Leistung auf die Fliesseigenschaften der Modellsubstanz ($\alpha$-D-Lactose Monohydrat, $d_{50}$ = 5.5 $\mu$m) auswirkt. Der Aufbau des Reaktors, sowie der Ablauf des Behandlungsprozesses sind identisch mit denjenigen aus dem Anwendungsbeispiel 1, mit der Ausnahme, dass die Vorwärtsleistung des RF-Generators variiert wird.

- Für diese Parameterstudie werden die RF-Vorwärtsleistungen von 50 W, 100 W und 200 W eingestellt. Die Kapazitäten des Anpassungsnetzwerkes werden so eingestellt, dass die reflektierte Leistung < 10 W beträgt. Damit werden effektive Leistungen von > 40 W, > 90 W und > 190 W erzeugt.

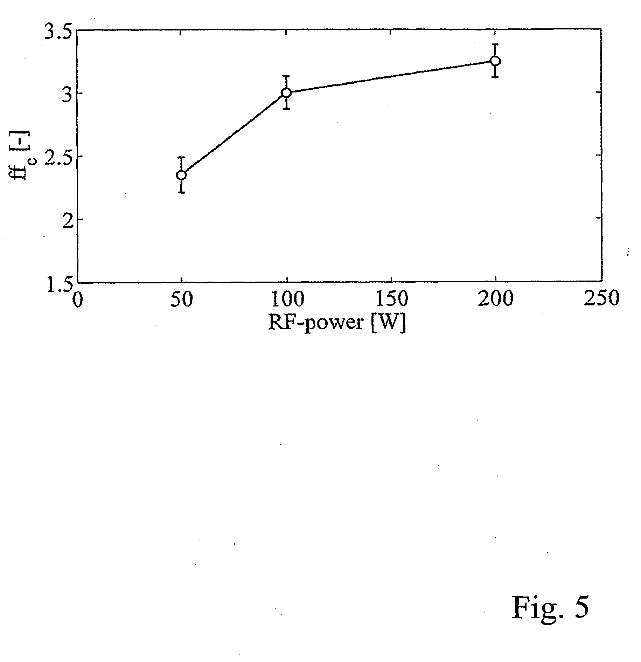

Die Fliessfähigkeiten der behandelten Pulver werden mit dem im Anwendungsbeispiel 1 beschriebenen Verfahren gemessen. Aus Abbildung 5 ist erkennbar, dass die Fliessfähigkeit mit zunehmender RF-Leistung zunimmt. Die höhere Leistung führt zu einer grösseren Fragmentierung des Monomers und somit zu einer erhöhten Abscheidungsrate. Die in Abbildung 5 dargestellten Werte beziehen sich auf die Vorwärtsleistung des RF-Generators.

BEZUGSZEICHENLISTE

| | |
|---|---|
| 1 | Steigrohr |
| 2 | Mikrowellenplasmaquelle |
| 3 | Druckbehälter |
| 4 | Massendurchflussregler |
| 5 | Verdampfungsmodul |
| 6 | Massendurchflussregler |
| 7 | Massendurchflussregler |
| 8 | Sinterplatte |
| 9 | kapazitive Drucksonde |
| 10 | Messung Druckabfall |
| 11 | gekrümmter Einlauf |
| 12 | Zyklonabscheider |
| 13 | Staubfilter |
| 14 | Wälzkolbenpumpe |
| 15 | Drehschieberpumpe |
| 16 | Fallrohr |
| 17 | Thermoelement |
| 18 | Massendurchflussregler |
| 19 | Fluidisierungszone |
| 20 | Vorratsbehälter |
| 21 | Förderschnecke |
| 22 | Fallrohr |
| 23 | Elektroden |
| 24 | Generator |
| 25 | Anpassungsnetzwerk |
| 26 | Auffangbehälter |
| 27 | Zyklon |

**Patentansprüche**

1.  Verfahren zur Bildung von Nanopartikeln und deren Anlagerung an Substratpartikel, **dadurch gekennzeichnet dass** ein Gasstrom durch eine Plasmazone geführt wird, in welcher eine elektrische Gasentladung zur Erzeugung eines anisothermen Plasmas, insbesondere zur Erzeugung von freien Ladungsträgern und angeregten neutralen Spezies, verwendet wird, wobei dem Gasstrom vor, in oder nach der Plasmazone ein gasförmiges Monomer beigemischt wird, welches als Ausgangsmaterial für die chemische Reaktion zur Bildung der Nanopartikel dient, und wobei die freien Ladungsträger und angeregten neutralen Spezies direkt in der Plasmazone oder nach der Plasmazone dazu verwendet werden, das gasförmige Monomer in einen chemisch reaktiven Zustand und zu einer homogenen chemischen Reaktion zu bringen, so dass sich durch chemische Abscheidung aus der Gasphase Nanopartikel bilden, und dass die gebildeten Nanopartikel in einer Behandlungszone, durch welche ein Substratpartikel- und/oder Gas-Substratpartikel-Strom unter Einfluss der Gasströmung und/oder der Gewichtskraft geführt wird, an die Oberfläche der Substratpartikel durch die Kollision der beiden Partikelarten angelagert werden.

2.  Verfahren nach Anspruch 1, **dadurch gekennzeichnet, dass** in einem Gas-Substratpartikelstrom unter Einfluss der Gasströmung und der Gewichtskraft die Substratpartikel durch eine Behandlungszone geführt werden, wobei der Gasstrom neben den Substratpartikeln ein gasförmiges Monomer enthält, welches als Ausgangsmaterial für die chemische Reaktion zur Bildung der Nanopartikel dient; dass in der Behandlungszone eine elektrische Gasentladung zur Erzeugung eines anisothermen Plasmas als Plasmazone verwendet wird, wobei die freien Elektronen resp. die Ladungsträger und angeregten neutralen Spezies dazu verwendet werden, das gasförmige Monomer in einen chemisch reaktiven Zustand und zu einer homogenen chemischen Reaktion zu bringen, so dass sich durch chemische Abscheidung aus der Gasphase Nanopartikel bilden; und dass die gebildeten Nanopartikel direkt innerhalb der Plasmazone an die Oberfläche der Substratpartikel durch die Kollision der beiden Partikelarten angelagert werden.

3.  Verfahren nach Anspruch 1, **dadurch gekennzeichnet, dass** Plasmazone und Behandlungszone räumlich zusammenfallen oder dass die Behandlungszone der Plasmazone im wesentlichen unmittelbar nachfolgt, wobei sich in letzterem Fall bevorzugtermassen der Gasstrom aus der Plasmazone und der Substratpartikelstrom kreuzen.

4.  Verfahren nach einem der Ansprüche 1-3, **dadurch gekennzeichnet, dass** die Substratpartikel ein einziges Mal durch die Behandlungszone geführt werden, wobei es sich bei der Behandlungszone vorzugsweise um ein Fallrohr oder ein Steigrohr handelt.

5.  Verfahren nach einem der vorhergehenden Ansprüche, **dadurch gekennzeichnet, dass** die Substratpartikel in der Behandlungszone verweilen, wobei es sich bei der Behandlungszone vorzugsweise um einen Trommelreaktor oder eine Wirbelschicht handelt.

6.  Verfahren nach einem der vorhergehenden Ansprüche, **dadurch gekennzeichnet, dass** die Feststoffpartikel mehrmals z.B. periodisch durch die Behandlungszone geführt werden, wobei es sich bei der Behandlungszone bevorzugtermassen um ein Steigrohr einer zirkulierenden Wirbelschicht handelt.

7.  Verfahren gemäss einem der vorhergehenden Ansprüche, **dadurch gekennzeichnet, dass** die Substratpartikel und der Gasstrom an unterschiedlichen Stellen im Reaktor eingespeist werden.

8.  Verfahren gemäss einem der vorhergehenden Ansprüche, **dadurch gekennzeichnet, dass** ein flüssiges Monomer verwendet wird, insbesondere bevorzugt in Form eines Aerosols.

9.  Verfahren nach einem der vorhergehenden Ansprüche, **dadurch gekennzeichnet, dass** die chemische Reaktion über mehrere Reaktionsstufen abläuft.

10. Verfahren nach einem der vorhergehenden Ansprüche, **dadurch gekennzeichnet, dass** die Nanopartikel untereinander kollidieren und agglomerieren, bevor sie sich an der Substratoberfläche anlagern und/oder dass die Nanopartikel auf der Substratoberfläche mit anderen Nanopartikeln kollidieren und agglomerieren.

11. Verfahren nach einem der vorhergehenden Ansprüche, **dadurch gekennzeichnet, dass** die freien, noch nicht angelagerten Nanopartikel, durch heterogene chemische Abscheidung aus der Gasphase beschichtet werden und/oder dass die bereits an der Substratoberfläche angelagerten Nanopartikel durch heterogene chemische Abscheidung aus der Gasphase beschichtet werden.

12. Verfahren nach einem der vorhergehenden Ansprüche, **dadurch gekennzeichnet, dass** die, noch nicht oder nur unwesentlich durch Nanopartikel beladene Substratoberfläche durch heterogene chemische Abscheidung aus der Gasphase beschichtet wird, und/oder dass die Substratoberfläche ausschliesslich durch heterogene chemische Abscheidung aus der Gasphase beschichtet wird.

13. Verfahren nach einem der vorhergehenden Ansprüche, **dadurch gekennzeichnet, dass** zur Erzeugung einer elektrischen Gasentladung eine Mikrowellenkopplung, Mittel- oder Hochfrequenzkopplung oder DC-Anregung verwendet wird.

14. Verfahren nach einem der vorhergehenden Ansprüche, **dadurch gekennzeichnet, dass** es sich beim Monomer um HDMSO oder eine Mischung enthaltend HDMSO handelt.

15. Verfahren nach einem der vorhergehenden Ansprüche, **dadurch gekennzeichnet, dass** in der Plasmazone ein anisothermes Niederdruckplasma oder ein Normaldruckplasma vorliegt.

16. Verfahren nach Anspruch 15, **dadurch gekennzeichnet, dass** das Niederdruckplasma bei einem Druck im Bereich von 0.27 mbar bis 2.7, mbar betrieben wird.

17. Verfahren nach einem der vorhergehenden Ansprüche, **dadurch gekennzeichnet, dass** das Monomer in einem Gasstrom, insbesondere bevorzugt in einem Inertgasstrom, mit einem Gehalt auf den Systemdruck bezogen im Bereich von- 10 % zugeführt wird, insbesondere bevorzugt im Bereich von 2 - 5 %.

18. Verfahren nach einem der vorhergehenden Ansprüche, **dadurch gekennzeichnet, dass** Substratpartikel mit einer mittleren Grösse im Bereich von 500nm -500 $\mu$m, insbesondere bevorzugt im Bereich von 5 $\mu$m-500 $\mu$m in den Prozess eingeführt werden, wobei die Substratpartikel bevorzugtermassen elektrisch nicht leitend sind.

19. Verfahren nach einem der vorhergehenden Ansprüche, **dadurch gekennzeichnet, dass** es sich bei den Substratpartikeln um Partikel handelt, welche bis zu einer Temperatur von wenigstens 70°C stabil sind, wobei es sich insbesondere bevorzugt um pharmazeutische wirksame Komponenten handelt.

20. Verfahren nach einem der vorhergehenden Ansprüche, **dadurch gekennzeichnet, dass** die Nanopartikel eine mittlere Grösse im Bereich von weniger als 500 nm aufweisen und dass die untere Grenze der Nanopartikelgrösse durch die entsprechende Molekülgrösse der im Prozess abgeschiedenen Substanz gegeben ist, wobei ein Bereich von 0.5 nm-0.5 $\mu$m für die mittlere Grösse bevorzugt ist.

21. Verfahren nach einem der vorhergehenden Ansprüche, **dadurch gekennzeichnet, dass** die mittlere, im Fall von periodischer Führung akkumulierte Verweilzeit in der Behandlungszone im Bereich von 10 ms - 1 s liegt.

22. Verwendung eines Verfahrens nach einem der vorhergehenden Ansprüche zur Erhöhung der Fliessfähigkeit von Substratpartikeln.

23. Vorrichtung zur Durchführung eines Verfahrens nach einem der vorhergehenden Ansprüche, **dadurch gekennzeichnet, dass** eine Plasmazone vorhanden ist, durch welche ein Gasstrom geführt wird, und in welcher eine elektrische Gasentladung zur Erzeugung eines anisothermen Plasmas, insbesondere zur Erzeugung von freien Ladungsträgern und angeregten neutralen Spezies, verwendet wird, wobei dem Gasstrom vor, in oder nach der Plasmazone ein gasförmiges Monomer beigemischt wird, welches als Ausgangsmaterial für die chemische Reaktion zur Bildung der Nanopartikel dient, und wobei die freien Ladungsträger und angeregten neutralen Spezies direkt in der Plasmazone oder nach der Plasmazone dazu verwendet werden, das gasförmige Monomer in einen chemisch reaktiven Zustand und zu einer homogenen chemischen Reaktion zu bringen, so dass sich durch chemische Abscheidung aus der Gasphase Nanopartikel bilden, und dass eine Behandlungszone vorhanden ist, durch welche ein Substratpartikel- und/oder Substratpartikel-Gas-Strom unter Einfluss der Gasströmung und/oder der Gewichtskraft geführt wird, und in welcher die gebildeten Nanopartikel an die Oberfläche der Substratpartikel durch die Kollision der beiden Partikelarten angelagert werden.

24. Vorrichtung nach Anspruch 23, **dadurch gekennzeichnet, dass** ein erstes Führungselement, vorzugsweise in Form eines Rohres, angeordnet ist, in welchem die Substratpartikel im Sinne eines Fallrohres oder eines Steigrohres geführt werden, und dass ein zweites, vorzugsweise im wesentlichen in einem rechten Winkel, zum ersten Führungselement angeordnetes und in dieses erste Führungselement mündendes zweites Führungselement vorhanden ist, in welchem zweiten Führungselement der Gasstrom mit den Monomeren geführt ist und in welchem zweiten Rohr die anisotherme Plasmazone angeordnet ist, sodass im wesentlichen unmittelbar hinter dieser Plasmazone die dort gebildeten Nanopartikel im Gasstrom an die Oberfläche der Substratpartikel durch die Kollision der beiden Partikelarten angelagert werden.

25. Vorrichtung nach einem der vorhergehenden Ansprüche 23 oder 24, **dadurch gekennzeichnet, dass** der beschriebene Prozess in einer Vorrichtung, vorzugsweise in einer Strahlmühle integriert ist.

**26.** Vorrichtung nach einem der Ansprüche 23 - 25, **dadurch gekennzeichnet, dass** Plasmazone und Behandlungszone räumlich zusammenfallen oder dass die Behandlungszone der Plasmazone im wesentlichen unmittelbar nachfolgt, wobei sich in letzterem Fall bevorzugtermassen der Gasstrom aus der Plasmazone und der Substratpartikelstrom kreuzen.

**Claims**

**1.** Method for producing nano particles and their attachment to substrate particles,
**characterized in that**
a gas stream is guided through a plasma zone, in which an electric gas discharge is used for the production of an non-isothermal plasma, especially for the generation of free charge carriers and excited neutral species, wherein a gaseous monomer, which serves as a starting material for the chemical reaction for the formation of the nano particles, is admixed to the gas stream before, in or after the plasma zone, and wherein the free charge carriers and excited neutral species are used directly in the plasma zone or after the plasma zone, in order to bring the gaseous monomer into a chemically reactive state and to a homogenous chemical reaction, such that nano particles are formed by chemical separation from the gaseous phase, and
**in that**, in a treatment zone through which a substrate particle- and/or gas-substrate particle stream is guided under the influence of the gas stream and/or gravity, the formed nano particles are attached to the surface of the substrate particles by the collision of the two particle types.

**2.** Method according to claim 1, **characterized in that** the substrate particles are guided through a treatment zone in a gas-substrate particle stream under the influence of the gas stream and gravity, wherein the gas stream contains, in addition to the substrate particles, a gaseous monomer, which serves as a starting material for the chemical reaction for the formation of the nano particles; that in the treatment zone an electric gas discharge is used as a plasma zone for the generation of a non-isothermal plasma, wherein the free electrons or the charge carriers and excited neutral species, respectively, are used in order to bring the gaseous monomer into a chemically reactive state and to a homogenous chemical reaction, such that nano particles are formed by chemical separation from the gaseous phase; and that, directly within the plasma zone, the formed nano particles are attached to the surface of the substrate particles by the collision of the two particle types.

**3.** Method according to claim 1, **characterized in that** the plasma zone and the treatment zone are spatially congruent, or that the treatment zone is located immediately following the plasma zone, wherein in the latter case, preferably the gas stream from the plasma zone and the substrate particle stream intersect.

**4.** Method according to one of claims 1-3, **characterized in that** the substrate particles are guided through the treatment zone one single time, wherein the treatment zone preferably is a down pipe or a riser pipe.

**5.** Method according to one of the preceding claims, **characterized in that** the substrate particles remain in the treatment zone, wherein the treatment zone preferably is a drum reactor or a fluidized bed.

**6.** Method according to one of the preceding claims, **characterized in that** the solid particles are guided several times, e.g. periodically, through the treatment zone, wherein the treatment zone preferably is a riser pipe of a circulating fluidized bed.

**7.** Method according to one of the preceding claims, **characterized in that** the substrate particles and the gas stream are fed at different locations in the reactor.

**8.** Method according to one of the preceding claims, **characterized in that** a fluid monomer is used, especially preferably in the form of an aerosol.

**9.** Method according to one of the preceding claims, **characterized in that** the chemical reaction proceeds over several reaction steps.

**10.** Method according to one of the preceding claims, **characterized in that** the nano particles collide and agglomerate with eachother, prior to attaching to the substrate surface and/or that the nano particles collide and agglomerate with other nano particles on the substrate surface.

**11.** Method according to one of the preceding claims, **characterized in that** the free, not yet attached nano particles are coated by heterogenous chemical separation from the gaseous phase, and/or that the nano particles already attached to the substrate surface are coated by heterogenous chemical separation from the gaseous phase.

**12.** Method according to one of the preceding claims, **characterized in that** the substrate surface which is not yet or only insignificantly loaded with nano particles is coated by heterogenous chemical separation from the gaseous phase, and/or that the substrate surface is coated exclusively by heterogeneous chemical separation from the gaseous phase.

13. Method according to one of the preceding claims, **characterized in that** for the generation of an electric gas discharge a microwave coupling, a middle- or high frequency coupling or DC-excitation is used.

14. Method according to one of the preceding claims, **characterized in that** the monomer is HDMSO or a mixture containing HDMSO.

15. Method according to one of the preceding claims, **characterized in that** the plasma zone contains a non-isothermal low pressure plasma or a normal pressure plasma.

16. Method according to claim 15, **characterized in that** the low pressure plasma is operated at a pressure in the range of 0.27 mbar to 2.7 mbar.

17. Method according to one of the preceding claims, **characterized in that** the monomer is introduced in a gas stream, especially preferably in an inert gas stream, in an amount in the range of 1-10% in relation to the system pressure, especially preferably in the range of 2-5%.

18. Method according to one of the preceding claims, **characterized in that** substrate particles with an average particle size in the range of $500m$-$500\mu m$, especially preferably in the range of $5\mu m$-$500\mu m$, are introduced into the process, wherein the substrate particles preferably are electrically non-conductive.

19. Method according to one of the preceding claims, **characterized in that** the substrate particles are particles which are stable up to a temperature of at least 70°C, wherein they especially preferably are pharmaceutically active components.

20. Method according to one of the preceding claims, **characterized in that** the nano particles have an average size in the range of less than 500nm and that the bottom limit of the nano particle size is determined by the corresponding molecule size of the substance deposited during the process, wherein a range of 0.5nm-$0.5\mu m$ is preferred for the average size.

21. Method according to one of the preceding claims, **characterized in that** the average, in case of periodical guidance accumulated retention time in the treatment zone is in the range of 1 0ms-1 s.

22. Use of a method according to one of the preceding claims, for the increase of the flowability of substrate particles.

23. Device for carrying out a method according to one of the preceding claims, **characterized in that** a plasma zone is provided, through which a gas stream is guided, and in which an electric gas discharge is used for the generation of a non-isothermal plasma, specially for the generation of free charge carriers and excited neutral species, wherein a gaseous monomer, which is used as a starting material for the chemical reaction for the formation of nano particles, is admixed to the gas stream before, in or after the plasma zone, and wherein the free charge carriers and excited neutral species are used directly in the plasma zone or after the plasma zone in order to bring the gaseous monomer to a chemically reactive state and to a homogenous chemical reaction, such that nano particles are formed by chemical separation from the gaseous phase, and **in that** a treatment zone is provided, through which a substrate particle- and/or substrate particle gas stream is guided under the influence of the gas stream and/or gravity, and in which the formed nano particles are attached to the surface of the substrate particles by the collision of both particle types.

24. Device according to claim 23, **characterized in that** a first guiding element, preferably in the form of a pipe, is provided, in which the substrate particles are guided in the sense of a down pipe or a riser pipe, and that a second guiding element, preferably arranged in an essentially right angle to the first guiding element and opening out into this first guiding element, is provided, wherein in said second guiding element the gas stream is guided with the monomers, and wherein in said second pipe the non-isothermal plasma zone is located, such that essentially immediately behind this plasma zone, the nano particles formed therein are attached in the gas stream to the surface of the substrate particles by the collision of the two particle types.

25. Device according to of the preceding claims 23 or 24, **characterized in that** the described process is integrated in a device, preferably in a fluid energy mill

26. Device according to one of claims 23-25, **characterized in that** the plasma zone and the treatment zone are spatially congruent, or that the treatment zone is located essentially immediately following the plasma zone, wherein in the latter case preferably the gas stream from the plasma zone and the substrate particle stream intersect.

**Revendications**

1. Procédé pour former des nanoparticules et les déposer sur des particules de substrat, **caractérisé en ce que** un écoulement de gaz est passé dans une zone de plasma dans laquelle une décharge électrique dans un gaz est utilisée pour former un plasma anisotherme, en particulier pour former des porteurs de charge libres et une substance neutre excitée, l'écoulement de gaz étant mélangé avant, dans ou après la zone de plasma à un monomère gazeux qui sert de matériau de départ pour la réaction chimique de formation des nanoparticules, les porteurs de charge libres et la substance neutre excitée étant utilisés directement dans la zone de plasma ou après la zone de plasma pour amener le monomère gazeux dans un état chimiquement réactif et à réagir de chimiquement de manière homogène de telle sorte que des nanoparticules se forment par dépôt chimique depuis la phase gazeuse et

**en ce que** les nanoparticules formées sont déposées à la surface des particules de substrat par la collision des deux types de particules dans une zone de traitement par laquelle un écoulement de particules de substrat et/ou de particules de substrat et de gaz est passé sous l'influence de l'écoulement de gaz et/ou de la gravité.

2. Procédé selon la revendication 1, **caractérisé en ce que** les particules de substrat sont passées dans une zone de traitement dans un écoulement de particules de substrat et de gaz sous l'influence de l'écoulement de gaz et de la gravité, l'écoulement de gaz contenant en plus des particules de substrat un monomère gazeux qui sert de matériau de départ pour la réaction chimique de formation des nanoparticules, **en ce que** dans la zone de traitement, on utilise une décharge électrique dans un gaz pour former comme zone de plasma un plasma anisotherme, les électrons libres et/ou les porteurs de charge et la substance neutre excitée étant utilisés pour amener le monomère gazeux dans un état chimiquement réactif et à réagir chimiquement de manière homogène de manière à former des nanoparticules par dépôt chimique depuis la phase gazeuse, et **en ce qu'**à l'intérieur de la zone de plasma, les nanoparticules formées sont déposées directement à la surface des particules de substrat par collisions entre les deux types de particules.

3. Procédé selon la revendication 1, **caractérisé en ce que** la zone de plasma et la zone de traitement coïncident spatialement ou **en ce que** la zone de traitement suit essentiellement directement la zone de plasma, et dans ce dernier cas, l'écoulement de gaz qui sort de la zone de plasma et l'écoulement de particules de substrat se croisent de préférence.

4. Procédé selon l'une des revendications 1 à 3, **caractérisé en ce que** les particules de substrat sont passées une seule fois dans la zone de traitement, la zone de traitement étant de préférence un tube descendant ou un tube montant.

5. Procédé selon l'une des revendications précédentes, **caractérisé en ce que** les particules de substrat séjournent dans la zone de traitement, la zone de traitement étant de préférence un réacteur en tambour ou un lit fluidisé.

6. Procédé selon l'une des revendications précédentes, **caractérisé en ce que** les particules de solide sont passées plusieurs fois, par exemple périodiquement, dans la zone de traitement, la zone de traitement étant de préférence un tube montant d'un lit fluidisé circulant.

7. Procédé selon l'une des revendications précédentes, **caractérisé en ce que** les particules de substrat et l'écoulement de gaz sont injectés dans le réacteur en différents endroits.

8. Procédé selon l'une des revendications précédentes, **caractérisé en ce qu'**on utilise un monomère qui présente de façon particulièrement préférable la forme d'un aérosol.

9. Procédé selon l'une des revendications précédentes, **caractérisé en ce que** la réaction chimique se déroule en plusieurs étapes.

10. Procédé selon l'une des revendications précédentes, **caractérisé en ce que** les nanoparticules entrent en collision mutuelle et s'agglomèrent avant de se déposer à la surface du substrat et/ou **en ce que** les nanoparticules entrent en collision avec d'autres nanoparticules et s'agglomèrent à la surface du substrat.

11. Procédé selon l'une des revendications précédentes, **caractérisé en ce que** les nanoparticules libres non encore déposées sont déposées par dépôt chimique hétérogène depuis la phase gazeuse et/ou **en ce que** les nanoparticules déjà déposées à la surface du substrat sont déposées par dépôt chimique hétérogène depuis la phase gazeuse.

12. Procédé selon l'une des revendications précédentes, **caractérisé en ce que** la surface du substrat qui n'est pas encore chargée ou n'est pas essentiellement chargée de nanoparticules est revêtue par dépôt chimique hétérogène depuis la phase gazeuse et/ou **en ce que** la surface du substrat est revêtue exclusivement par dépôt chimique hétérogène depuis la phase gazeuse.

13. Procédé selon l'une des revendications précédentes, **caractérisé en ce que** pour former une décharge électrique dans un gaz, on recourt à une injection des micro-ondes, à une injection de fréquences moyennes ou hautes ou à une excitation en courant continu.

**14.** Procédé selon l'une des revendications précédentes, **caractérisé en ce que** le monomère est le HDMSO ou un mélange qui contient du HDMSO.

**15.** Procédé selon l'une des revendications précédentes, **caractérisé en ce que** la zone de plasma contient un plasma anisotherme à basse pression ou un plasma sous pression normale.

**16.** Procédé selon la revendication 15, **caractérisé en ce que** le plasma à basse pression fonctionne à une pression comprise dans la plage de 0,27 mbar à 2,7 mbars.

**17.** Procédé selon l'une des revendications précédentes, **caractérisé en ce que** le monomère est apporté dans un écoulement de gaz et de manière particulièrement préférable dans un écoulement de gaz inerte, à une teneur de l'ordre de 1 à 10 % et de façon particulièrement préférable de l'ordre de 2 à 5 % sous la pression du système.

**18.** Procédé selon l'une des revendications précédentes, **caractérisé en ce que** l'on apporte dans le processus des particules de substrat d'une taille moyenne de l'ordre de 500 nm à 500 $\mu$m et de manière particulièrement préférable de l'ordre de 5 $\mu$m à 500 $\mu$m, les particules de substrat étant de préférence électriquement non conductrices.

**19.** Procédé selon l'une des revendications précédentes, **caractérisé en ce que** les particules de substrat sont des particules stables jusqu'à une température d'au moins 70°C, ces particules étant de façon particulièrement préférable des composants pharmaceutiquement actifs.

**20.** Procédé selon l'une des revendications précédentes, **caractérisé en ce que** les nanoparticules ont une taille moyenne de l'ordre de moins de 500 nm et **en ce que** la limite inférieure de la taille des nanoparticules est définie par la taille correspondante des molécules de la substance déposée dans le processus, une plage de 0,5 nm à 0,5 $\mu$m étant préférée pour la taille moyenne.

**21.** Procédé selon l'une des revendications précédentes, **caractérisé en ce que** le temps de séjour moyen dans la zone de traitement est de l'ordre de 10 ms à 1 s, ce temps de séjour étant le temps de séjour cumulé dans le cas d'une amenée périodique.

**22.** Utilisation d'un procédé selon l'une des revendications précédentes pour augmenter la fluidité de particules de substrat.

**23.** Dispositif en vue de l'exécution d'un procédé selon l'une des revendications précédentes, **caractérisé en ce qu'**il présente une zone de plasma dans laquelle on fait passer un écoulement de gaz et dans laquelle une décharge électrique dans un gaz est utilisée pour former un plasma anisotherme et en particulier pour former des porteurs de charge libres et une substance neutre excitée, un monomère gazeux qui sert de matériau de départ pour la réaction chimique de formation des nanoparticules étant ajouté à l'écoulement de gaz avant, dans ou après la zone de plasma, les porteurs de charge libres et la substance neutre excitée étant utilisés directement dans la zone de plasma ou après la zone de plasma pour amener le monomère gazeux dans un état chimiquement réactif et à réagir chimiquement de manière homogène de telle sorte que des nanoparticules se forment par dépôt chimique depuis la phase gazeuse et

**en ce qu'**il présente une zone de traitement par laquelle on fait passer un écoulement de particules de substrat et/ou un écoulement de particules de substrat et de gaz sous l'influence de l'écoulement de gaz et/ou de la gravité et dans laquelle les nanoparticules formées se déposent à la surface de particules de substrat suite aux collisions entre les deux types de particules.

**24.** Dispositif selon la revendication 23, **caractérisé en ce qu'**il présente un premier élément de guidage, de préférence sous la forme d'un tube, dans lequel les particules de substrat sont passées dans le sens d'un tube descendant ou d'un tube montant et **en ce qu'**il présente un deuxième élément de guidage disposé de préférence essentiellement à angle droit par rapport au premier élément de guidage et débouchant dans ce premier élément de guidage, l'écoulement de gaz contenant les monomères étant passé dans le deuxième élément de guidage, la zone de plasma anisotherme étant disposée dans le deuxième tube de telle sorte que les nanoparticules formées dans cette zone de plasma se déposent à la surface des particules de substrat essentiellement directement en aval de cette zone de plasma dans l'écoulement de gaz, suite aux collisions entre les deux types de particules.

**25.** Dispositif selon l'une des revendications 23 ou 24 qui précèdent, **caractérisé en ce que** le processus décrit est intégré dans un dispositif et de préférence dans un pulvérisateur à jets.

**26.** Dispositif selon l'une des revendications 23 à 25, **caractérisé en ce que** la zone de plasma et la zone de traitement coïncident spatialement ou **en ce que** la zone de traitement suit la zone de plasma essentiellement directement, et dans ce dernier cas, l'écoulement de gaz provenant de la zone de plasma et l'écoulement de particules de substrat se croisent de préférence.

Fig. 1

Fig. 2

Fig. 3

Fig. 4

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CA 2492129
- WO 2004007594 A
- US 2005118094 A
- JP 2004024953 B
- EP 0807461 A
- US 5620743 A
- US 4685419 A
- US 5234723 A
- US 2004182293 A
- US 5925325 A

**In der Beschreibung aufgeführte Nicht-Patentliteratur**

- **I. Zimmermann ; M. Eber ; K. Meyer.** Nanomaterials as Flow Regulators in Dry Powders. *Z. Phys. Chem.,* 2004, vol. 218, 51-102
- **S. Jonat ; S. Hasenzahl ; A. Gray ; P.C. Schmidt.** Mechanism of Glidants: Investigation of the Effect of Different Collodial Silicon Dioxide Types on Powder Flow by Atomic Force and Scanning Electron Microscopy. *J. Pharm. Sci.,* 2004, vol. 93 (10), 2635-2644
- **J.H. Werth ; M. Linsenbühler ; S.M. Dammer ; Z. Farkas ; H. Hinrichsen ; K.-E. Wirth ; D.E. Wolf.** Agglomeration of Charged Nanopowders in Suspensions. *Powder Technol.,* 2003, vol. 133, 106-112
- An Introduction to Plasma Polymerization. **N. Morosoff.** Plasma Deposition, Treatment, and Etching of Polymers. Academy Press, 1990
- **A. Grill.** Cold Plasma in Materials Fabrication, From Fundamentals to Applications. IEEE Press, 1994
- **R.M. Young ; E. Pfender.** Generation and Behavior of Fine Particles in Thermal Plasmas - a Review. *Plasma Chem. Plasma Process,* 1985, vol. 5 (1), 1-37
- **N. Rao ; S. Girshick ; J. Heberlein ; P. McMurry ; S. Jones ; D. Hansen ; B. Micheel.** Nanoparticle Formation Using a Plasma Expansion Process. *Plasma Chem. Plasma Process,* 1995, vol. 15 (4), 581-606
- **G.S. Selwyn ; J. Singh ; R.S. Bennett.** In situ laser diagnostic studies of plasma-generated particulate contamination. *J. Vac. Sci. Technol.,* 1989, vol. A 7 (4), 2758-2765
- **H. Kersten ; G. Thieme ; M. Fröhlich ; D. Bojic ; D.H. Tung ; M. Quaas ; H. Wulff ; R. Hippler.** Complex (dusty) plasmas: Examples for applications and observation of magnetron-induced phenomena. *Pure Appl. Chem.,* 2005, vol. 77 (2), 415-428
- **T. Fujimoto ; K. Okuyama ; M. Shimada ; Y. Fujishige ; M. Adachi ; I. Matsui.** Particle generation and thin film surface morphology in the tetraethylorthosilicate/oxygen plasma enhanced chemical vapor deposition process. *J. Appl. Phys.,* 2000, vol. 99 (5), 3047-3052
- **A. Bouchoule ; A. Plain ; L. Boufendi ; J.Ph. Blondeau ; C. Laure.** Particle generation and behavior in silane-argon low-pressure discharge under continuous or pulsed-frequency excitation. *J. Appl. Phys.,* 1991, vol. 70 (4), 1991-2000
- **J.H. Chu, L. I.** Fine silicon oxide particles in rf hollow magnetron discharges. *J. Appl. Phys.,* 1993, vol. 74 (7), 4741-4745
- **S. Schlabach ; V. Szabo ; D. Vollath ; A. Braun ; R. Clasen.** Structure of Alumina and Zirconia Nanoparticles Synthesized by the Karlsruhe Microwave Plasma Process. *Solid State Phenomena,* 2004, vol. 99-100, 191-196
- **J.W. Kim ; Y.S. Kim ; H.S. Choi.** Thermal characteristics of surface-crosslinked high density polyethylene beads as a thermal energy storage material. *Korean J. Chem. Eng.,* 2003, vol. 19 (4), 632-637
- **M. Karches ; Ch. Bayer ; Ph. Rudolf von Rohr.** A circulating fluidised bed for plasma-enhanced chemical vapor deposition on powders at low temperatures. *Surf. Coat. Tech.,* 1999, vol. 119, 879-885
- **Ch. Bayer ; M. Karches ; A. Matthews ; Ph. Rudolf von Rohr.** Plasma Enhanced Chemical Vapor Deposition on Powders in a Low Temperature Plasma Fluidized Bed. *Chem. Eng. Technol.,* 1998, vol. 21 (5), 427-430
- **C. Arpagaus ; A. Sonnenfeld ; Ph. Rudolf von Rohr.** A Downer Reactor for Short-time Plasma Surface Modification of Polymer Powders. *Chem. Eng. Technol.,* 2005, vol. 28 (1), 87-94

- **A. Bouchoule.** Dusty Plasmas, Physics, Chemistry and Technological Impacts in Plasma Processing. Wiley, 1999
- **D. Schulze.** Zur Fliessfähigkeit von Schüttgütern - Definition und Messverfahren. *Chem. Ing. Tech.,* 1995, vol. 67 (1), 60-68
- **D. Schulze ; A. Wittmaier.** Flow Properties of Highly Dispersed Powders at Very Small Consolidation Stresses. *Chem. Eng. Technol.,* 2003, vol. 26 (2), 133-137